# EUROPEAN PATENT SPECIFICATION

(11) **EP 2 705 365 B1**
(45) Date of publication and mention of the grant of the patent: **13.07.2016**
(21) Application number: 12705431.0
(22) Date of filing: 31.01.2012
(51) Int. Cl.: G01N 33/543, G01N 33/53

(54) **IMMUNOASSAY FOR DIRECT DETERMINATION OF ANTIGEN CONTENT OF PRODUCTS COMPRISING ADJUVANT-COUPLED-ANTIGEN PARTICLES**
IMMUNOASSAY ZUR DIREKTEN BESTIMMUNG DES ANTIGENGEHALTS VON PRODUKTEN MIT AN HILFSSTOFFE GEKOPPELTEN ANTIGENTEILCHEN
IMMUNOESSAI POUR LA DÉTERMINATION DIRECTE DE LA TENEUR EN ANTIGÈNE DE PRODUITS COMPRENANT DES PARTICULES D'ANTIGÈNE COUPLÉ À UN ADJUVANT

(43) Date of publication of application: 12.03.2014
(73) Proprietor: Hal Allergy Holding B.V., 2333 CH Leiden (NL)
(72) Inventor: KERKVLIET, Erica, Helena, Maria, 2332 XB Leiden (NL)
(74) Representative: van Kooij, Adriaan
(86) International application number: PCT/IB2012/050447
(87) International publication number: WO 2012/095834

(56) References cited:
- WO-A2-01/51926
- US-A1- 2005 069 868
- KATZ J B ET AL: "IN VITRO ASSESSMENT OF VIRAL ANTIGEN CONTENT IN INACTIVATED ALUMINUM HYDROXIDE ADJUVANTED VACCINES", JOURNAL OF VIROLOGICAL METHODS, ELSEVIER BV, NL, vol. 25, no. 1, 1 January 1989 (1989-01-01), pages 101-108, XP001009902, ISSN: 0166-0934, DOI: 10.1016/0166-0934(89)90104-3
- R. VAN REE ET AL: "EU Forum: The CREATE Project: development of certified reference materials for allergenic products and validation of methods for their quantification", ALLERGY, vol. 63, no. 3, 1 March 2008 (2008-03-01), pages 310-326, XP55024944, ISSN: 0105-4538, DOI: 10.1111/j.1398-9995.2007.01612.x
- ZHU ET AL.: JOURNAL OF IMMUNOLOGICAL METHODS, vol. 344, 2009, pages 73-78, XP026057801, cited in the application

## Description

The present invention relates to methods for the direct determination of the antigen potency of products comprising adjuvant-coupled-antigen particles.

To ensure the quality of products comprising antigen-coupled-adjuvant particles, and especially antigen-coupled-aluminium particles, it is essential to determine the amount, identity and/or integrity of the antigens bound to the adjuvant, and especially aluminium, particles.

Determining the quality of these products is particularly relevant after formulation, after storage and/or immediately before administration to ensure accurate dosing of the antigen in combination with the adjuvant.

Although multiple analysis techniques are available to determine the amount of an antigen in solution such as conventional ELISA, these techniques are not suitable to accurately and reproducibly determine the amount, identity and/or integrity of adjuvant-coupled-antigen particles because of, amongst others, of the presence aggregates in the product.

Considering the above, it is an object of the present invention, amongst other objects, to provide a method capable of easily, accurately and/or reproducibly determining the potency, i.e. immunogenic potential, of products comprising antigen-coupled-adjuvant particles such as vaccines and other immunotherapeutic agents.

A key factor determining the ability to directly, easily, accurately and/or reproducibly determine the potency, i.e. immunogenic potential, of products comprising antigen-coupled-adjuvant particles is to able to determine a dose-response curve of the product. Dose-response curves allow, for example, determining the 50% IgG inhibition being a reliable measure for the immunogenic potency of the product.

WO 01/51926 discloses a competitive enzyme immunoassay for assessing total antigen content of aluminium adsorbed antigens.

The above objects, amongst other objects, are met by the present invention through a method as defined in the appended claim 1.

Especially, the above objects, amongst other objects, are met by the present invention through a method for determining the antigen potency of a product comprising antigen-coupled-adjuvant particles, the method comprises the steps of:
a) contacting the product comprising antigen-coupled-adjuvant particles with immunoglobulin molecules capable of recognizing the antigen under conditions allowing antigen-immunoglobulin binding;
b) providing a surface with the antigen, without the adjuvant, immobilized thereon;
c) contacting the immunoglobulin contacted product of step (a) with the surface of step (b) under conditions allowing antigen-immunoglobulin binding;
d) removing non-bound immunoglobulin molecules and antigen-coupled-adjuvant particles bound immunoglobulin molecules;
e) detecting antigen-bound immunoglobulin molecules thereby directly determining the antigen content of a product comprising antigen-coupled-adjuvant particles
wherein the antigen-coupled-adjuvant particles are antigen-coupled-aluminium particles or antigen-coupled-tyrosine particles and wherein the immunoglobulin is IgG.

The present inventors have surprisingly discovered that detecting antigen-bound immunoglobulin molecules provides a reliable, accurate and/or reproducible measure of the antigen content in the original product. In other words, the present inventors have surprisingly discovered that the amount of antigen-bound immunoglobulin molecules detected by the present method is inversely proportional to the antigen content in the original product.

Present step (c) is preferably preceded, after step (a), by a centrifugation step pelleting the antigen-coupled-adjuvant particles.

According to a preferred embodiment of the present method, the product is a vaccine or an immunotherapeutic agent.

The present antigen-coupled-adjuvant particles are antigen-coupled-aluminium particles. Currently, the only adjuvants approved for human vaccine are aluminium comprising adjuvants.

Generally, the aluminium based adjuvants used in human vaccines are based on aluminium hydroxide, Alhydrogel, aluminium phosphate, potassium aluminium sulphate and alum. Accordingly, according to yet another preferred embodiment of the present invention, the aluminium is selected from the group consisting of aluminium hydroxide, Alhydrogel, aluminium phosphate, potassium aluminium sulphate and alum.

Because the present invention is particularly beneficial in particulate vaccines or other immunogenic medicaments, the present antigen is preferably an allergen or allergoid.

The immunoglobulin used for detection is an IgG immunoglobulin such as a polyclonal or monoclonal antibody.

Preferably, step (e) of the present method comprises contacting the antigen-bound immunoglobulin molecules with a second immunoglobulin molecule capable of recognizing the antigen-bound immunoglobulin molecule under conditions allowing antigen-bound immunoglobulin molecule - second immunoglobulin molecule binding and detecting antigen-bound immunoglobulin molecule - second immunoglobulin molecule binding.

The present second immunoglobulin molecule is preferably provided with a detectable label, preferably, an enzyme, more preferably HRP. The use of an enzyme, and especially HRP, provides signal amplification through conversion of a detectable substrate thereby providing increased sensitivity of detection.

The present surface is preferably provided by an ELISA plate allowing efficient and automated use of the present method. In other words, the present method is preferably performed in one or more ELISA plate wells allowing efficient immobilization of the antigen to the surface, efficient handling of one or more steps of the present method such as removal non-bound immunoglobulin molecules and antigen-coupled-adjuvant particles bound immunoglobulin molecules and/or the subsequent detection step.

According to a preferred embodiment of the present invention, the product comprising antigen-coupled-adjuvant particles is a suspension, and especially a suspension wherein the antigen-coupled-adjuvant particles are at least partially aggregated.

According to the present invention, step (d) preferably comprises washing the surface one or more times with a suitable wash solution such as a washing buffer.

The present invention will be further detailed in the following example of a particularly preferred embodiment of the present invention. In the example, reference is made to figures wherein:
- **Figure 1:**: shows a schematic representation of a method according to the present invention;
- **Figure 2:**: shows IgG inhibition curves of two batches of allergoids, an allergen preparation (native extract) and an aluminium adsorbed allergoid;
- **Figure 3:**: shows the reproducibility of the present method by analysing the potency of an allergoid sample along as a control. The other curves are three independently diluted alu-adsorbed allergoid preparations;
- **Figure 4:**: shows the result of a comparison of the present method with a prior art method designated as DAFIA.

### Example

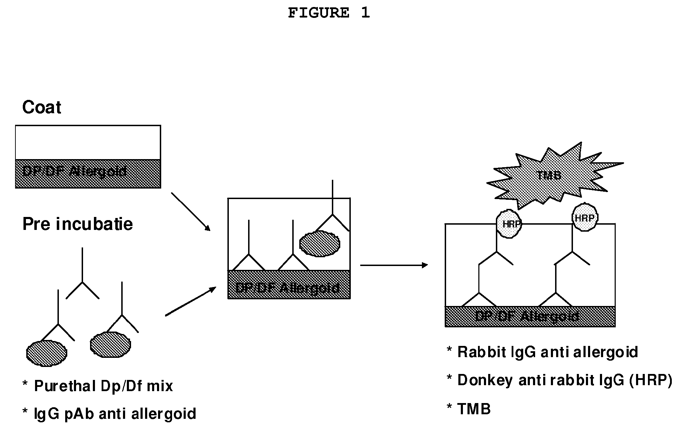

A novel method for the determination of the potency of aluminium adsorbed allergoids has been developed and validated. The present method is schematically outlined in **figure 1****.**

A potency assay is an IgG inhibition test and is based on the inhibition of IgG binding on allergoid-coated 96 wells plates by alu-adsorbed allergoids (drug product).

Briefly, 96 well microtiter plates are coated overnight with 1 µg/ml allergoid in 50 mM bicarbonate buffer, pH 9.6. After coating, the plates are washed and blocked with 3% BSA.

In parallel with the blocking step, rabbit anti-allergoid IgG polyclonal antibodies are pre-incubated with different concentrations of aluminium-adsorbed allergoids in 0.1 % BSA, TBS-Tween, pH 7.5.

After 2 hours, the mixtures of IgG and aluminium-adsorbed allergoid are added to the wells of the allergoid coated microtiter plate and free allergoid-specific IgG antibodies bind to the allergoid-coated plates.

Then, plates are washed and bound IgG is detected with HRP-labelled anti-rabbit antibodies. Finally, plates are washed, stained with TMB for exactly 15 minutes and the colouring is stopped with 0.5 M H₂SO₄. The colour intensity of the wells is measured at 450 nm using a microtiterplate reader.

The extent to which the IgG antibodies bind the plate in presence of aluminium-adsorbed allergoid is compared with the maximum amount of IgG antibodies binding the plate in the absence of aluminium-adsorbed allergoid (E-max value).

Results are finally expressed as percentage inhibition relative to the E-max. As potency parameter the 50% IgG inhibition value is taken. This value is calculated by plotting an inhibition curve using a 4-parameter logistic model and uses the 50% value on the curve.

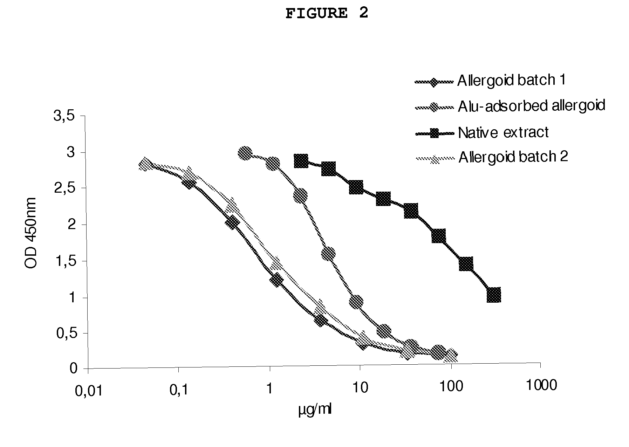

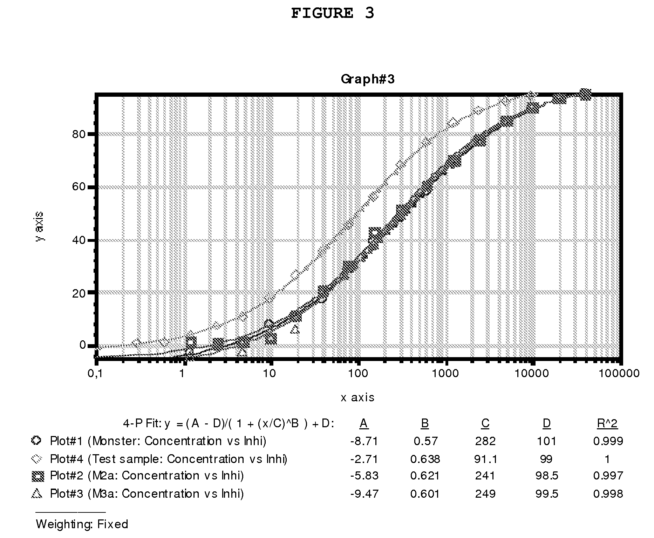

A representative result of the present method is shown in **figures 2** **and** **3****.**

Briefly, as is also schematically shown in **figure 1****,** allergoid specific rabbit IgG is pre-incubated with different concentrations of drug product. Subsequently, the mixture is incubated on allergoid coated 96 wells plates. Then, plates are washed and bound IgG is detected with HRP-labelled anti-rabbit antibodies. Finally, TMB substrate is added to create colour that is measured at 450 nm. The colour intensity is a measure for the amount of bound IgG. As potency parameter the 50% IgG inhibition value is taken. This value is calculated by plotting an inhibition curve using a 4-parameter logistic model and uses the 50% value on the curve.

### Comparative example

Before the development of the present method, it was investigated whether an assay described by Zhu et al. in the Journal of Immunological Methods, 344 (2009), pages 73 - 78 could be reproduced.

This assay, the DAFIA (Direct Alhydrogel Formulation Immunoassay), was designed to directly determine antigen content on aluminium. Based on the DAFIA, the following method was used.

Aluminium-adsorbed allergoid was added to U-bottom plates and washed by centrifugation with PBS, pH 7,4. Then, the plates were blocked with 3% BSA/PBS, and after washing incubated with biotin labelled anti-allergoid antibodies. After washing, HRP-labelled streptavidin was added and, after washing, stained with TMB.

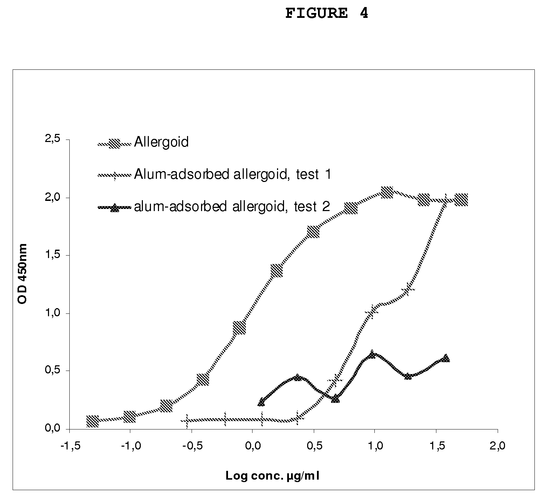

The results are presented in **figure 4****.** A non-adsorbed allergoid preparation was tested along the alum-adsorbed allergoids as test control.

Results: a number of tests were performed, however, the tests reveal highly variable results (an example is shown in the figure) and no proper dose-responses were found. Further experiments showed that the signal mainly came from unbound allergoid. The DAFIA was not suitable for measuring potency of aluminium-adsorbed allergoid.

## Claims

1. Method for determining the potency of a product comprising antigen-coupled-adjuvant particles, the method comprises the steps of:
a) contacting the product comprising antigen-coupled-adjuvant particles with immunoglobulin molecules capable of recognizing the antigen under conditions allowing antigen-immunoglobulin binding;
b) providing a surface with the antigen, without the adjuvant, immobilized thereon;
c) contacting the immunoglobulin contacted product of step (a) with the surface of step (b) under conditions allowing antigen-immunoglobulin binding;
d) removing non-bound immunoglobulin molecules and antigen-coupled-adjuvant particles bound immunoglobulin molecules;
e) detecting antigen-bound immunoglobulin molecules thereby determining the potency of a product comprising antigen-coupled-adjuvant particles by determining the 50% IgG inhibition using a dose-response curve;
wherein the antigen-coupled-adjuvant particles are antigen-coupled-aluminium particles or antigen-coupled-tyrosine particles and wherein the immunoglobulin is IgG.

2. Method according to claim 1, wherein the product is a vaccine or an immunotherapeutic agent.

3. Method according claim 1, wherein the aluminium is selected from the group consisting of aluminium hydroxide, Alhydrogel, aluminium phosphate, potassium aluminium sulphate and alum.

4. Method according to any of the claims 1 to 3, wherein the antigen is an allergen.

5. Method according to any of the claims 1 to 4, wherein step (e) comprises contacting the antigen-bound immunoglobulin molecules with a second immunoglobulin molecule capable of recognizing the antigen-bound immunoglobulin molecule under conditions allowing antigen-bound immunoglobulin molecule - second immunoglobulin molecule binding and detecting antigen-bound immunoglobulin molecule - second immunoglobulin molecule binding.

6. Method according to claim 5, wherein the second immunoglobulin comprises a detectable label.

7. Method according to claim 6, wherein the label is an enzyme, preferably HRP.

8. Method according to any of the claims 1 to 7, wherein the surface is provided by an ELISA plate.

9. Method according to any of the claims 1 to 8 wherein the product comprising antigen-coupled-adjuvant particles is a suspension.

10. Method according to any of the claims 1 to 9, wherein step (d) comprises washing the surface one or more times.

## Patentansprüche

1. Verfahren zum Bestimmen der Wirksamkeit eines Produkts, welches Antigen-gekoppelte Adjuvanzpartikel umfasst, wobei das Verfahren die Schritte umfasst:
a) Kontaktieren des Produkts, welches Antigen-gekoppelte Adjuvanzpartikel umfasst, mit Immunglobulinmolekülen, die zum Erkennen des Antigens unter Bedingungen, welche Antigen-Immunglobulin-Binden erlauben, fähig sind;
b) Bereitstellen einer Oberfläche mit dem Antigen, welches darauf immobilisiert ist, ohne das Adjuvanz;
c) Kontaktieren des mit Immunglobulin in Kontakt gebrachten Produkts des Schritts (a) mit der Oberfläche des Schritts (b) unter Bedingungen, welche Antigen-Immunglobulin-Binden erlauben;
d) Entfernen nicht-gebundener Immunglobulinmoleküle und Antigen-gekoppelte Adjuvanzpartikel-gebundener Immunglobulinmoleküle;
e) Detektieren von Antigen-gebundenen Immunglobulinmolekülen, dadurch Bestimmen der Wirksamkeit eines Produkts, welches Antigen-gekoppelte Adjuvanzpartikel umfasst, durch Bestimmen der 50% IgG-Inhibition unter Verwendung einer Dosis-Wirkungs-Kurve;
wobei die Antigen-gekoppelten Adjuvanzpartikel Antigen-gekoppelte Aluminiumpartikel oder Antigen-gekoppelte Tyrosinpartikel sind und wobei das Immunglobulin IgG ist.

2. Verfahren gemäß Anspruch 1, wobei das Produkt ein Impfstoff oder ein immuntherapeutisches Agens ist.

3. Verfahren gemäß Anspruch 1, wobei das Aluminium ausgewählt ist aus einer Gruppe bestehend aus Aluminiumhydroxid, Alhydrogel, Aluminiumphosphat, Kaliumaluminiumsulfat und Alaun.

4. Verfahren gemäß einem der Ansprüche 1 bis 3, wobei das Antigen ein Allergen ist.

5. Verfahren gemäß einem der Ansprüche 1 bis 4, wobei Schritt (e) umfasst Kontaktieren des Antigen-gebundenen Immunglobulinmoleküls mit einem zweiten Immunglobulinmolekül, welches zum Erkennen des Antigen-gebundenen Immunglobulinmoleküls unter Bedingungen, die das Binden von Antigen-gebundenem Immunglobulinmolekül - zweites Immunglobulinmolekül erlauben, fähig ist und Detektieren des Bindens von Antigen-gebundenem Immunglobulinmolekül - zweites Immunglobulinmolekül.

6. Verfahren gemäß Anspruch 5, wobei das zweite Immunglobulin eine detektierbare Markierung umfasst.

7. Verfahren gemäß Anspruch 6, wobei die Markierung ein Enzym ist, vorzugsweise HRP.

8. Verfahren gemäß einem der Ansprüche 1 bis 7, wobei die Oberfläche durch eine ELISA-Platte bereitgestellt wird.

9. Verfahren gemäß einem der Ansprüche 1 bis 8, wobei das Produkt, welches Antigen-gekoppelte Adjuvanzpartikel umfasst, eine Suspension ist.

10. Verfahren gemäß einem der Ansprüche 1 bis 9, wobei der Schritt (d) ein- oder mehrmaliges Waschen der Oberfläche umfasst.

## Revendications

1. Procédé pour déterminer la puissance d'un produit comprenant des particules d'adjuvant couplées à un antigène, le procédé comprenant les étapes consistant à :
a) mettre le produit comprenant des particules d'adjuvant couplées à un antigène en contact avec des molécules d'immunoglobuline capables de reconnaître l'antigène dans des conditions permettant une liaison antigène-immunoglobuline ;
b) mettre à disposition une surface sur laquelle l'antigène, sans l'adjuvant, est immobilisé ;
c) mettre le produit de l'étape (a), ayant été mis en contact avec des immunoglobulines, en contact avec la surface de l'étape (b) dans des conditions permettant une liaison antigène-immunoglobuline ;
d) éliminer les molécules d'immunoglobuline non liées et les molécules d'immunoglobuline liées aux particules d'adjuvant couplées à l'antigène ;
e) détecter les molécules d'immunoglobuline liées à l'antigène en déterminant ainsi la puissance d'un produit comprenant des particules d'adjuvant couplées à un antigène par la détermination de l'inhibition des IgG à 50 % en utilisant une courbe de dose-réponse ;
où les particules d'adjuvant couplées à un antigène sont des particules d'aluminium couplées à un antigène ou des particules de tyrosine couplées à un antigène, et où l'immunoglobuline est une IgG.

2. Procédé selon la revendication 1, dans lequel le produit est un vaccin ou un agent d'immunothérapie.

3. Procédé selon la revendication 1, dans lequel l'aluminium est sélectionné dans le groupe consistant en l'hydroxyde d'aluminium, l'Alhydrogel, le phosphate d'aluminium, le sulfate d'aluminium et de potassium et l'alun.

4. Procédé selon l'une quelconque des revendications 1 à 3, dans lequel l'antigène est un allergène.

5. Procédé selon l'une quelconque des revendications 1 à 4, dans lequel l'étape (e) comprend la mise en contact des molécules d'immunoglobulines liées à un antigène avec une seconde molécule d'immunoglobuline capable de reconnaître la molécule d'immunoglobuline liée à un antigène dans des conditions permettant une liaison molécule d'immunoglobuline liée à un antigène-seconde molécule d'immunoglobuline et la détection d'une liaison molécule d'immunoglobuline liée à un antigène-seconde molécule d'immunoglobuline.

6. Procédé selon la revendication 5, dans lequel la seconde immunoglobuline comprend un marqueur détectable.

7. Procédé selon la revendication 6, dans lequel le marqueur est une enzyme, de préférence la HRP.

8. Procédé selon l'une quelconque des revendications 1 à 7, dans lequel la surface est mise à disposition par une plaque ELISA.

9. Procédé selon l'une quelconque des revendications 1 à 8, dans lequel le produit comprenant des particules d'adjuvant couplées à un antigène est une suspension.

10. Procédé selon l'une quelconque des revendications 1 à 9, dans lequel l'étape (d) comprend un lavage de la surface une ou plusieurs fois.
